# EUROPEAN PATENT SPECIFICATION

(11) **EP 4 277 565 B1**
(45) Date of publication and mention of the grant of the patent: **30.04.2025**
(21) Application number: 22705987.0
(22) Date of filing: 15.02.2022
(51) Int. Cl.: A61B 90/00

(54) **FLEXIBLE ELECTRONIC CIRCUIT FOR ULTRASOUND CATHETERS**
FLEXIBLE ELEKTRONISCHE SCHALTUNG FÜR ULTRASCHALLKATHETER
CIRCUIT ÉLECTRONIQUE FLEXIBLE POUR CATHÉTERS À ULTRASONS

(30) Priority: 05.03.2021 US 202163157232 P
(43) Date of publication of application: 22.11.2023
(73) Proprietor: St. Jude Medical, Cardiology Division, Inc., St. Paul MN 55117 (US)
(72) Inventor: LUPOTTI, Fermin, Lake Forest, CA 92630 (US); BLANCK, Arthur G., Ramona, CA 92065 (US)
(74) Representative: Mathys & Squire
(86) International application number: PCT/US2022/016402
(87) International publication number: WO 2022/186978

(56) References cited:
- WO-A2-2007/105009
- WO-A2-2007/105009
- US-A1- 2003 029 907
- US-A1- 2003 029 907
- US-A1- 2018 295 711
- US-A1- 2018 295 711
- US-B1- 9 213 361
- US-B1- 9 213 361
- PHYSIK INSTRUMENTS: "Piezo Components with Flexible Printed Circuit Boards", 13 August 2019 (2019-08-13), pages 1 - 1, XP055921191, Retrieved from the Internet <URL:https://www.youtube.com/watch?v=_DTBhh6FpgQ> [retrieved on 20220513]
- PHYSIK INSTRUMENTS: "Piezo Components with Flexible Printed Circuit Boards", 13 August 2019 (2019-08-13), pages 1 - 1, XP055921191, Retrieved from the Internet <URL:https://www.youtube.com/watch?v=_DTBhh6FpgQ> [retrieved on 20220513]

## Description

### CROSS-REFERENCE TO RELATED APPLICATIONS

This application claims the benefit of United States provisional application no. 63/157,232, filed 5 March 2021.

### BACKGROUND

The present disclosure relates generally to catheters that are used in the human body. In particular, the present disclosure relates to a small form-factor flexible electronic circuit suitable for incorporation into an intravascular catheter, such as within the tip of such a catheter.

Catheters are used for an ever-growing number of procedures. For example, catheters are used for diagnostic, therapeutic, and ablative procedures, to name just a few examples. Typically, the catheter is manipulated through the patient's vasculature and to the intended site, for example, a site within the patient's heart.

It is known to incorporate various electronic components into the tips of intravascular catheters. For instance, piezoelectric ultrasound transducers and supported electronics packages (implemented, for example, as an application-specific integrated circuit (ASIC) or field-programmable gate array (FPGA)) may be mounted within the tip of an intracardiac echocardiography (ICE) catheter, such as disclosed in United States patent application publication no. 2018/0289356 and United States provisional application no. 62/987,574. The relatively small size of such components, however, increases the complexity, and therefore the cost, of manufacture.
WO 2007/105009 A2 discloses circuits comprising a substrate carrying a conductive track.
US 2018/0295711 A1 discloses a method for manufacturing an electronic assembly.
US 2003/0029907 A1 discloses a transfer film for use with a flexible circuit compression connector.

### BRIEF SUMMARY

Aspects of the invention are set out in the independent claims and preferred features are set out in the dependent claims. Disclosed herein is a flexible electronic circuit, including: a flexible substrate having a first surface and a second surface opposite the first surface, wherein the first surface includes a recess; a localization element disposed within the recess; and a conductive connector pad disposed on the flexible substrate.

The flexible substrate includes a first portion having a first thickness and a second portion having a second thickness thinner than the first thickness. The recess is positioned within the first portion of the flexible substrate, and the conductive connector pad may be disposed on the second portion of the flexible substrate.

The localization element may include a magnetic localization element and/or an impedance-based localization element.

In embodiments of the disclosure, there are a plurality of recesses and a corresponding plurality of localization elements respectively disposed within the plurality of recesses.

Aspects of the disclosure also include a thermistor mounted to the substrate, for example, within the recess.

Further aspects of the disclosure include an ultrasound transducer assembly mounted to the substrate, for example, on the second surface of the substrate.

Still further aspects of the disclosure include an adhesive overlaying at least a portion of the substrate.

Also disclosed herein is a tip assembly for an intravascular catheter. The tip assembly includes a shell; and a flexible electronic circuit disposed within the shell. In turn, the flexible electronic circuit includes: a flexible substrate having a first surface and a second surface opposite the first surface, wherein the first surface includes a recess; a localization element disposed within the recess; and a conductive connector pad disposed on the flexible substrate.

In embodiments of the tip assembly, the flexible electronic circuit includes a plurality of recesses and a corresponding plurality of localization elements respectively disposed within the plurality of recesses. The flexible electronic circuit may also include a thermistor and/or an ultrasound transducer assembly mounted to the flexible substrate. The thermistor may be mounted in a recess.

The instant disclosure also relates to an intravascular catheter, including an elongate catheter body terminating at a distal end; a tip assembly secured to the distal end of the elongate catheter body; and a flexible electronic circuit disposed within the tip assembly. The flexible electronic circuit includes: a flexible substrate having a first surface and a second surface opposite the first surface, wherein the first surface includes a recess; a localization element disposed within the recess; and a conductive connector pad disposed on the flexible substrate.

In embodiments of the intravascular catheter, the flexible electronic circuit includes a plurality of recesses and a corresponding plurality of localization elements respectively disposed within the plurality of recesses. The flexible electronic circuit may also include a thermistor and/or an ultrasound transducer assembly mounted to the flexible substrate. The thermistor may be mounted in a recess.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a perspective view of a representative catheter according to aspects of the instant disclosure.
Figure 2 schematically illustrates the distal region of the representative catheter of Figure 1, including the interior of the tip portion.
Figure 3A is a top view of a flexible electronic circuit according to aspects of the instant disclosure.
Figure 3B is a side view of a flexible electronic circuit according to aspects of the instant disclosure.
Figure 4 illustrates a flexible electronic circuit assembly according to embodiments disclosed herein.

While multiple embodiments are disclosed, still other embodiments of the present disclosure will become apparent to those skilled in the art from the following detailed description, which shows and describes illustrative embodiments. Accordingly, the drawings and detailed description are to be regarded as illustrative in nature and not restrictive.

### DETAILED DESCRIPTION

Aspects of the instant disclosure relate to flexible electronic circuits suitable for use to connect electronics packages integrated into the distal ends of intravascular catheters (*e.g*., positioned within the distal tip assemblies of such catheters). Those of ordinary skill in the art will appreciate that the teachings herein can be applied to good advantage in connection with various types of catheters, including, but not limited to, intracardiac echocardiography (ICE) catheters.

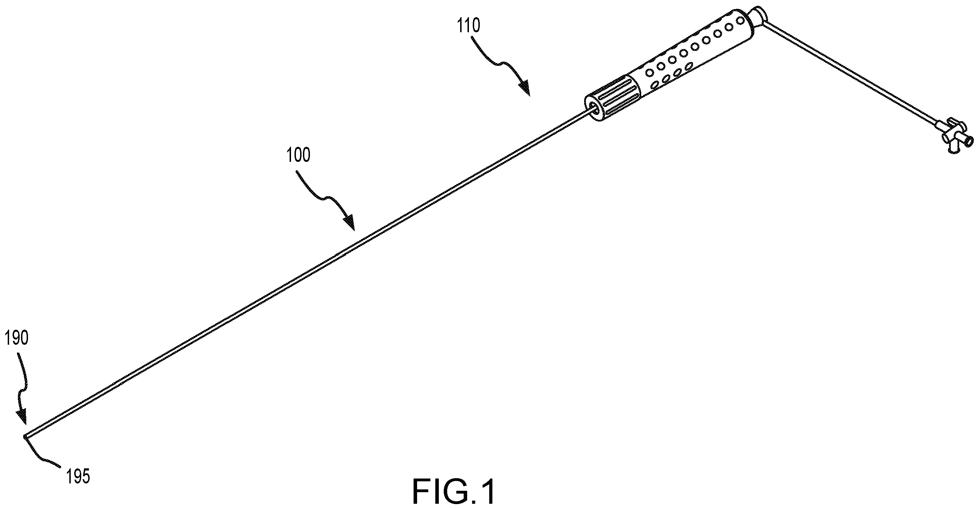

For purposes of illustration, Figure 1 depicts a perspective view of a representative catheter 100, including a shaft 105 having a proximal portion 110 and a distal portion 190, which terminates in a tip 195. Insofar as the basic construction of catheter 100 will be familiar to those of ordinary skill in the art, the details thereof will be omitted herein, except to the extent relevant to an understanding of the instant disclosure.

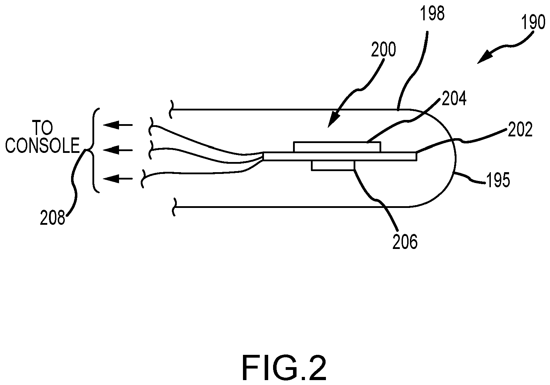

Figure 2 is a schematic representation of the interior of distal portion 190 of catheter 100, including tip 195. Tip 195 can include a shell 198 and a flexible electronic circuit 200 disposed within shell 198.

Flexible electronic circuit 200, in turn, includes a flexible substrate 202 and a transducer package (*e.g*., electronics package 204 and ultrasound transducer assembly 206) mounted to substrate 202. A wiring harness 208, including a plurality of wires, interconnects flexible electronic circuit 200 to an external device, such as an electroanatomical mapping system (*e.g.,* the EnSite Precision^{™} Cardiac Mapping System of Abbott Laboratories) or a catheter interface module. Only three wires are shown within wiring harness 208, but it should be understood that this is merely exemplary and that more or fewer wires may be used in a device according to the present teachings.

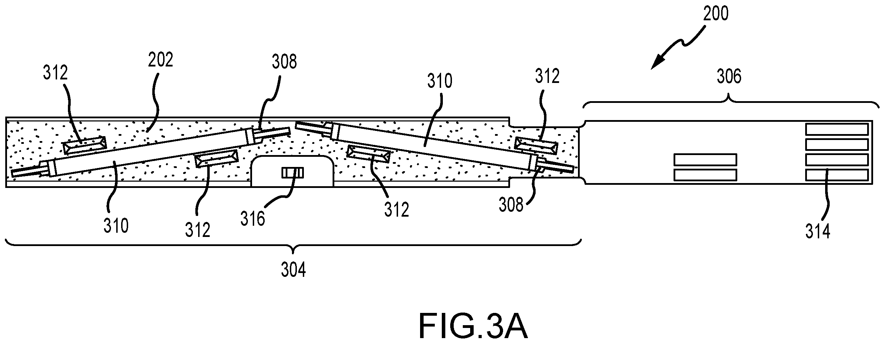

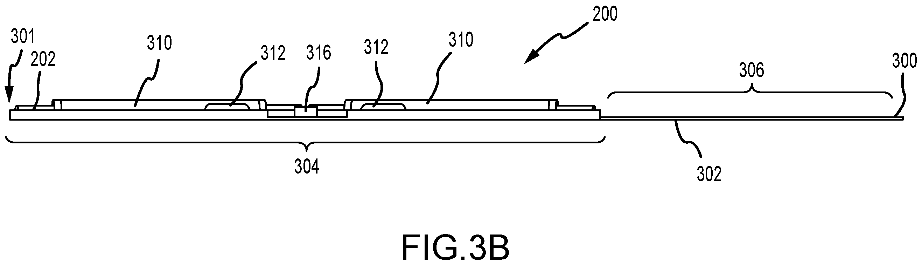

Various details of flexible electronic circuit 200 are shown in Figures 3A and 3B. In embodiments according to the invention, substrate 202 includes a first surface (visible in the plane of Figure 3A) and a second surface opposite the first surface. The thickness of substrate 202 at any given point is defined between the first and second surfaces at that point.

More particularly, and as best illustrated in Figure 3B, the thickness of substrate 202 can vary along its length. In embodiments according to the invention, substrate 202 includes a thicker first portion 304 (*e.g.,* to support localization elements) and a thinner second portion 306 (*e.g.,* to increase flexibility at the point of interconnection to wiring harness 208). First and second surfaces 301, 302, respectively, define the thickness of the thicker first portion 304 of substrate 202, while first and second surfaces 300, 302, respectively, define the thickness of the thinner second portion 306 of substrate 202. It is contemplated that the thicker first portion 304 of substrate 202 may be formed by bonding one or more additional flexible circuit layers to first surface 300 to build up the additional thickness.

First surface 301 of substrate 202 also includes a recess 308 (*e.g.,* a trench or groove) into the thickness of substrate 202. According to the invention, recess 308 is located within the thicker first portion 304 of substrate 202.

Recess 308 facilitates reliable and accurate positioning of components on substrate 202. In embodiments of the disclosure, a pair of localization elements 310 (*e.g.,* magnetic or impedance-based localization elements) are mounted to substrate 202 as part of electronics package 204 in a respective pair of recesses 308. For example, a pair of five degree of freedom (5DOF) magnetic localization elements 310, mounted at a slight angle with respect to each other within corresponding recesses 308, as shown in Figure 3A, creates a six degree of freedom (6DOF) localization sensor, as described in United States patent application publication no. 2018/0289356. Solder pads 312 provide electrical connection to localization elements 310.

One or more conductive connector pads 314 are disposed on substrate 202. Although Figure 3A depicts conductive connector pads 314 only on first surface 300 of substrate 202, this is merely exemplary and the ordinarily-skilled artisan will appreciate that conductive connector pads 314 may be placed elsewhere on substrate 202 without departing from the scope of the instant disclosure.

Conductive connector pads 314 provide points for interconnection with wiring harness 208. Conductive connectors pads 314 are also conductively coupled to localization elements 310 (*e.g.,* to solder pads 312), so as to carry signals, such as power and communications signals, to and from localization elements 310 and, as discussed in further detail below, a temperature sensing element. Insofar as conductive coupling between the attachment point for wiring harness 208 and flexible circuit components will be familiar to those of ordinary skill in the art, it need not be further described herein.

In embodiments of the disclosure, flexible electronic circuit 200 may include additional components. For example, Figures 3A and 3B depict a thermistor 316 mounted to substrate 202. According to aspects of the disclosure, thermistor 316 may be mounted within an additional recess analogous to recesses 308. This not only facilitates reliable and accurate positioning of thermistor 316, but also allows for a quicker thermal response by virtue of its proximity to ultrasound transducer assembly 206 through the correspondingly diminished thickness of substrate 202. Other components, such as power supplies, pre-amplifiers, multiplexors, imaging element drivers, additional localization elements, and the like, may also be mounted to substrate 202.

According to aspects of the disclosure, an adhesive may be applied overlaying at least a portion of first and/or second surfaces 300, 302 of substrate 202.

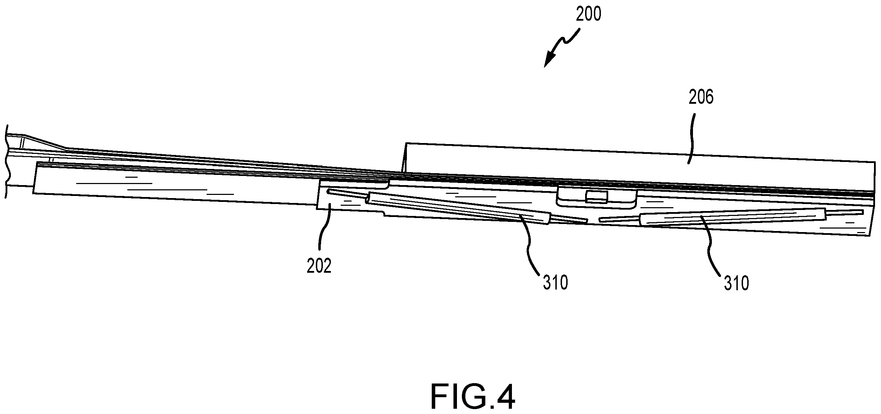

Figure 4 illustrates flexible electronic circuit 200, with localization elements 310 and ultrasound transducer assembly 206 mounted thereon for assembly into tip 195.

Although several embodiments have been described above with a certain degree of particularity, those skilled in the art could make numerous alterations to the disclosed embodiments without departing from the scope of this invention.

For example, rather than using individual wires, wiring harness 208 could utilize one or more long flex and/or printed circuits.

All directional references (*e.g.*, upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g.*, attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. A flexible electronic circuit (200) for a catheter, comprising:
a flexible substrate (202) having a first surface (301) and a second surface (302) opposite the first surface (301), wherein the first surface (301) includes a recess (308);
a localization element (310) disposed within the recess (308); and
a conductive connector pad (314) disposed on the flexible substrate (202) and conductively coupled to the localization element (310);
wherein the flexible substrate (202) comprises a first portion (304) having a first thickness and a second portion (306) having a second thickness thinner than the first thickness, wherein the recess (308) is positioned within the first portion of the flexible substrate (202).

2. The flexible electronic circuit (200) according to claim 1, wherein the conductive connector pad (314) is disposed on the second portion (306) of the flexible substrate (202).

3. The flexible electronic circuit (200) according to claim 1, wherein the localization element (310) comprises a magnetic localization element.

4. The flexible electronic circuit (200) according to claim 1, wherein the localization element (310) comprises an impedance-based localization element.

5. The flexible electronic circuit (200) according to claim 1, wherein:
the recess (308) comprises a plurality of recesses; and
the localization element (310) disposed within the recess (308) comprises a plurality of localization elements respectively disposed within the plurality of recesses.

6. The flexible electronic circuit (200) according to claim 1, further comprising a thermistor (316) mounted to the substrate (202).

7. The flexible electronic circuit (200) according to claim 6, wherein the thermistor is mounted to the flexible substrate (202) within the recess (308).

8. The flexible electronic circuit (200) according to claim 1, further comprising an ultrasound transducer assembly (206) mounted to the substrate (202).

9. The flexible electronic circuit (200) according to claim 8, wherein the transducer assembly (206) is mounted to the second surface (302) of the substrate (202).

10. The flexible electronic circuit (200) according to claim 1, further comprising an adhesive overlaying at least a portion of the substrate (202).

11. A tip assembly for an intravascular catheter, comprising:
a shell (198); and
the flexible electronic circuit (200) according to any one of claims 1 to 10, wherein the flexible electronic circuit (200) is disposed within the shell.

12. An intravascular catheter (100), comprising:
an elongate catheter body terminating at a distal end;
a tip assembly secured to the distal end of the elongate catheter body; and
the flexible electronic circuit (200) according to any one of claims 1 to 10, wherein the flexible electronic circuit (200) is disposed within the tip assembly.

## Patentansprüche

1. Eine flexible elektronische Schaltung (200) für einen Katheter, die Folgendes umfasst:
ein flexibles Substrat (202) mit einer ersten Oberfläche (301) und einer zweiten Oberfläche (302) gegenüber der ersten Oberfläche (301), wobei die erste Oberfläche (301) eine Aussparung (308) einschließt;
ein Lokalisationselement (310), das innerhalb der Aussparung (308) angeordnet ist; und
ein leitendes Anschluss-Pad (314), das auf dem flexiblen Substrat (202) angeordnet ist und leitend mit dem Lokalisationselement (310) gekoppelt ist;
wobei das flexible Substrat (202) einen ersten Abschnitt (304) mit einer ersten Dicke und einen zweiten Abschnitt (306) mit einer zweiten Dicke umfasst, die dünner ist als die erste Dicke, wobei die Aussparung (308) innerhalb des ersten Abschnitts des flexiblen Substrats (202) positioniert ist.

2. Flexible elektronische Schaltung (200) nach Anspruch 1, wobei das leitende Anschluss-Pad (314) auf dem zweiten Abschnitt (306) des flexiblen Substrats (202) angeordnet ist.

3. Flexible elektronische Schaltung (200) nach Anspruch 1, wobei das Lokalisationselement (310) ein magnetisches Lokalisationselement umfasst.

4. Flexible elektronische Schaltung (200) nach Anspruch 1, wobei das Lokalisationselement (310) ein impedanzbasiertes Lokalisationselement umfasst.

5. Flexible elektronische Schaltung (200) nach Anspruch 1, wobei:
die Aussparung (308) eine Vielzahl von Aussparungen umfasst; und
das Lokalisationselement (310), das innerhalb der Aussparung (308) angeordnet ist, eine Vielzahl von Lokalisationselementen umfasst, die jeweils innerhalb der Vielzahl von Aussparungen angeordnet sind.

6. Flexible elektronische Schaltung (200) nach Anspruch 1, die ferner einen an dem Substrat (202) angebrachten Thermistor (316) umfasst.

7. Flexible elektronische Schaltung (200) nach Anspruch 6, wobei der Thermistor an dem flexiblen Substrat (202) innerhalb der Aussparung (308) angebracht ist.

8. Flexible elektronische Schaltung (200) nach Anspruch 1, die ferner eine an dem Substrat (202) angebrachte Ultraschallwandlerbaugruppe (206) umfasst.

9. Flexible elektronische Schaltung (200) nach Anspruch 8, wobei die Wandlerbaugruppe (206) an der zweiten Oberfläche (302) des Substrats (202) angebracht ist.

10. Flexible elektronische Schaltung (200) nach Anspruch 1, die ferner einen Kleber umfasst, der über mindestens einem Abschnitt des Substrats (202) liegt.

11. Eine Spitzenbaugruppe für einen intravaskulären Katheter, der Folgendes umfasst:
eine Hülle (198); und
die flexible elektronische Schaltung (200) nach einem der Ansprüche 1 bis 10, wobei die flexible elektronische Schaltung (200) innerhalb der Hülle angeordnet ist.

12. Ein intravaskulärer Katheter (100), der Folgendes umfasst:
einen länglichen Katheterkörper, der an einem distalen Ende endet;
eine Spitzenbaugruppe, die an dem distalen Ende des länglichen Katheterkörpers befestigt ist; und
die flexible elektronische Schaltung (200) nach einem der Ansprüche 1 bis 10, wobei die flexible elektronische Schaltung (200) innerhalb der Spitzenbaugruppe angeordnet ist.

## Revendications

1. Circuit électronique flexible (200) pour un cathéter, comprenant :
un substrat flexible (202) présentant une première surface (301) et une deuxième surface (302) opposée à la première surface (301), dans lequel la première surface (301) inclut un creux (308) ;
un élément de localisation (310) disposé au sein du creux (308) ; et
un plot de connexion conducteur (314) disposé sur le substrat flexible (202) et couplé de manière conductrice à l'élément de localisation (310) ;
dans lequel le substrat flexible (202) comprend une première partie (304) ayant une première épaisseur et une deuxième partie (306) ayant une deuxième épaisseur plus fine que la première épaisseur, dans lequel le creux (308) est positionné au sein de la première partie du substrat flexible (202).

2. Circuit électronique flexible (200) selon la revendication 1, dans lequel le plot de connexion conducteur (314) est disposé sur la deuxième partie (306) du substrat flexible (202).

3. Circuit électronique flexible (200) selon la revendication 1, dans lequel l'élément de localisation (310) comprend un élément de localisation magnétique.

4. Circuit électronique flexible (200) selon la revendication 1, dans lequel l'élément de localisation (310) comprend un élément de localisation basé sur l'impédance.

5. Circuit électronique flexible (200) selon la revendication 1, dans lequel :
le creux (308) comprend une pluralité de creux ; et
l'élément de localisation (310) disposé au sein du creux (308) comprend une pluralité d'éléments de localisation disposés respectivement au sein de la pluralité de creux.

6. Circuit électronique flexible (200) selon la revendication 1, comprenant en outre une thermistance (316) montée sur le substrat (202).

7. Circuit électronique flexible (200) selon la revendication 6, dans lequel la thermistance est montée sur le substrat flexible (202) au sein du creux (308).

8. Circuit électronique flexible (200) selon la revendication 1, comprenant en outre un ensemble de transducteur à ultrasons (206) monté sur le substrat (202).

9. Circuit électronique flexible (200) selon la revendication 8, dans lequel l'ensemble de transducteur (206) est monté sur la deuxième surface (302) du substrat (202).

10. Circuit électronique flexible (200) selon la revendication 1, comprenant en outre un adhésif recouvrant au moins une partie du substrat (202).

11. Ensemble d'embout pour un cathéter intravasculaire, comprenant :
une coque (198) ; et
le circuit électronique flexible (200) selon l'une quelconque des revendications 1 à 10, dans lequel le circuit électronique flexible (200) est disposé au sein de la coque.

12. Un cathéter intravasculaire (100), comprenant :
un corps de cathéter allongé se terminant par une extrémité distale ;
un ensemble d'embout fixé à l'extrémité distale du corps de cathéter allongé ; et
le circuit électronique flexible (200) selon l'une quelconque des revendications 1 à 10, dans lequel le circuit électronique flexible (200) est disposé au sein de l'ensemble d'embout.
